(19) Europäisches Patentamt
European Patent Office
Office européen des brevets

(11) Veröffentlichungsnummer: **0 029 522**
**B1**

(12) # EUROPÄISCHE PATENTSCHRIFT

(45) Veröffentlichungstag der Patentschrift: **20.06.84**

(51) Int. Cl.³: **C 07 D 401/14,** C 08 K 5/34 // (C07D401/14, 211/58, 251/70)

(21) Anmeldenummer: **80106702.6**

(22) Anmeldetag: **31.10.80**

(54) **s-Triazinderivate, ihre Herstellung aus Halogentriazinylaminen und Polyaminen, und ihre Verwendung als Stabilisatoren.**

(30) Priorität: **06.11.79 DE 2944729**

(43) Veröffentlichungstag der Anmeldung: **03.06.81 Patentblatt 81/22**

(45) Bekanntmachung des Hinweises auf die Patenterteilung: **20.06.84 Patentblatt 84/25**

(84) Benannte Vertragsstaaten: **AT BE CH DE FR GB IT LI NL SE**

(56) Entgegenhaltungen:
**EP - A - 0 002 754**
**EP - A - 0 003 542**
**EP - A - 0 013 682**
**EP - A - 0 022 080**
**DE - A - 2 422 335**
**DE - A - 2 821 579**
**DE - A - 2 840 960**
**US - A - 3 925 376**
**US - A - 4 031 092**
**US - A - 4 161 592**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Wiezer, Hartmut, Dr., Hans-Fischer-Strasse 6, D-8906 Gersthofen (DE)**
Erfinder: **Pfahler, Gerhard, Dr., Karlsbader Strasse 27, D-8900 Augsburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

EP 0 029 522 B1

**Beschreibung**

Aus der EP-A1 13 682 ist bekannt, dass bestimmte Bis-triazinyl-diamine Ausgangsmaterialien für die Synthese hochmolekularer Triazinlichtschutzmittel darstellen, woraus abzuleiten ist, dass die Amine selbst als Stabilisatoren nur unzureichend geeignet sind. Triazinylamine, in welchen Aminreste durch Hydroxiethyl oder $-CH_2CH_2O\underset{\underset{O}{\|}}{C}-$Alkyl substituiert sind, beinhaltet die EP-A1 22 080. In der EP-A1 2 754 werden schliesslich durch Methylol- oder Alkoximethylgruppen substituierte Triazinylamine als besonders geeignete Stabilisatoren, die wegen dieser funktionellen Reste zu Reaktionen mit den zu stabilisierenden Kunststoffen befähigt sind, beschrieben. Hierdurch soll eine geringere Flüchtigkeit und Migration erreicht werden, jedoch gibt diese Reaktivität auch Anlass zur Selbstkondensation und den damit verbundenen Begleiterscheinungen wie Gasentwicklung, Vertrüben und auch Verfärben.

Gegenstand der vorliegenden Erfindung sind neue Triazinverbindungen, die sich von den in vorstehend genannten Druckschriften dadurch unterscheiden, dass sie andersartige Polyaminkomponenten enthalten und andere funktionelle Substituenten tragen, ein Verfahren zu ihrer Herstellung und ihre Verwendung als Lichtschutzmittel für organische Polymere.

Die neuen Triazinverbindungen lassen sich durch die allgemeine Formel (I) charakterisieren

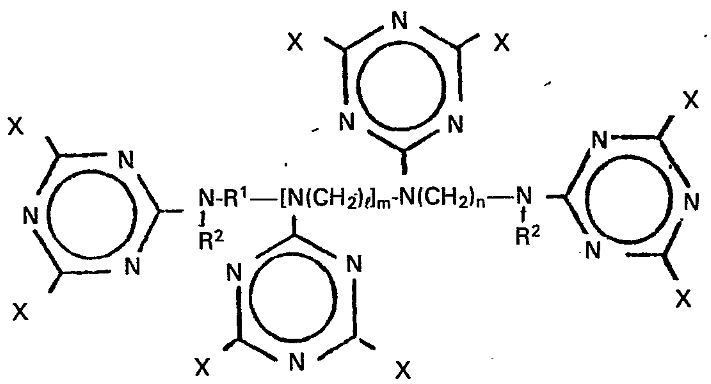

(I)

in der

$R^2$ für Wasserstoff, eine $C_1$- bis $C_6$-Alkylgruppe oder eine Gruppe der Formel (II)

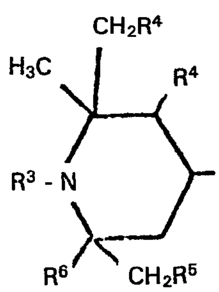

(II)

steht, in welcher

$R^3$ Wasserstoff oder $C_1$- bis $C_{18}$-Alkyl, vorzugsweise Wasserstoff oder $C_1$- bis $C_4$-Alkyl und insbesondere Wasserstoff ist,

$R^4$ und $R^5$ entweder gleich sind und Wasserstoff oder eine $C_1$- bis $C_5$-Alkylgruppe, vorzugsweise Wasserstoff oder eine Methylgruppe und insbesondere Wasserstoff bedeuten, wobei dann

$R^6$ eine Methylgruppe ist, oder

$R^4$ Wasserstoff oder $C_1$- bis $C_5$-Alkyl ist und

$R^5$ und $R^6$ zusammen mit den Kohlenstoffatomen, an die sie gebunden sind, einen $C_5$- oder $C_6$-Cycloalkylring oder eine Gruppe der Formel

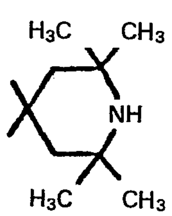

darstellen

die Bedeutung einer Gruppe der Formel (III)

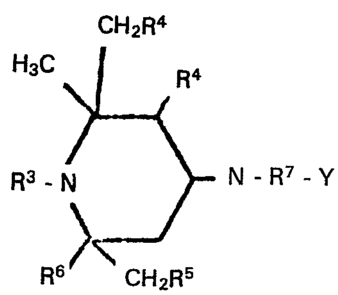

(III)

hat, in welcher $R^3$, $R^4$, $R^5$ und $R^6$ die bereits angegebene Bedeutung haben,

$R^7$ für eine Alkylengruppe mit 2 bis 4 C-Atomen, die durch eine Methylgruppe substituiert sein kann, und

Y für eine Gruppe der Formeln $-OR^8$ oder $-N(R^9)_2$ steht, mit $R^8$ = $C_1$- bis $C_{18}$-Alkyl und $R^9$ = Methyl oder Ethyl, und

$R^1$ wenn m eine ganze Zahl von 0 bis 3 ist, eine Alkylengruppe der Formel $-(CH_2)_r-$ bedeutet, wobei im Falle von m = 0 die Indices r und n gleich oder verschieden sein können und für eine ganze Zahl von 2 bis 6, vorzugsweise 2 oder 3, stehen, im Falle von m = 1, 2 oder 3 jedoch der Index $\ell$ für 2 oder 3 steht und die Indices r sowie n gleich sind und ebenfalls die Bedeutung von 2 oder 3 haben.

Beispiele für die neuen Verbindungen sind:

1. N,N',N''-Tris-{2,4-[N-(2,2,6,6-tetramethyl-4-

-piperidyl)-3-dimethylaminopropylamino]-1,3,5-triazin-6-yl}-diethylentriamin

2. N,N',N''-Tris-{2,4-[N-(2,2,6,6-tetramethyl-4-piperidyl)-3-diethylaminopropylamino]-1,3,5-triazin-6-yl}-diethylentriamin
3. N,N',N''-Tris-{2,4-[N-(2,2,6,6-tetramethyl-4-piperidyl)-3-dimethylaminopropylamino]-1,3,5-triazin-6-yl}-dipropylentriamin
4. N,N',N''-Tris-{2,4-[N-(2,2,6,6-tetramethyl-4-piperidyl)-3-diethylaminopropylamino]-1,3,5-triazin-6-yl}-dipropylentriamin
5. N,N',N''-Tris-{2,4-[N-(2,2,6,6-tetramethyl-4-piperidyl)-3-dimethylaminopropylamino]-1,3,5-triazin-6-yl}-3-(2-aminoethyl)-aminopropylamin
6. N,N',N''-Tris-{2,4-[N-(2,2,6,6-tetramethyl-4-piperidyl)-3-diethylaminopropylamino]-1,3,5-triazin-6-yl}-3-(2-aminoethyl)-aminopropylamin
7. N,N',N'',N'''-Tetrakis-{2,4-[N-(2,2,6,6-tetramethyl-4-piperidyl)-3-dimethylaminopropylamino]-1,3,5-triazin-6-yl}-triethylentetramin
8. N,N',N'',N'''-Tetrakis-{2,4-[N-(2,2,6,6-tetramethyl-4-piperidyl)-3-diethylaminopropylamino]-1,3,5-triazin-6-yl}-triethylentetramin
9. N,N',N'',N'''-Tetrakis-{2,4-[N-(2,2,6,6-tetramethyl-4-piperidyl)-3-dimethylaminopropylamino]-1,3,5-triazin-6-yl}-tripropylentetramin
10. N,N',N'',N'''-Tetrakis-{2,4-[N-(2,2,6,6-tetramethyl-4-piperidyl)-diethylaminopropylamino]-1,3,5-triazin-6-yl}-tripropylentetramin
11. N,N',N''-Tris-{2,4-[N-(2,2,6,6-tetramethyl-4-piperidyl)-dimethylaminopropylamino]-1,3,5-triazin-6-yl}-bis-(6-aminohexyl)-amin
12. N,N',N'',N'''-Tetrakis-{2,4-[N-(2,2,6,6-tetramethyl-4-piperidyl)-dimethylaminopropylamino]-1,3,5-triazin-6-yl}-4,7-diazadecan-1,10-diamin
13. N,N',N'',N'''-Tetrakis-{2,4-[N-(2,2,6,6-tetramethyl-4-piperidyl)-diethylaminopropylamino]-1,3,5-triazin-6-yl}-4,7-diazadecan-1,10-diamin
14. N,N',N'',N''',N$^{IV}$-Pentakis-{2,4-[N-(2,2,6,6-tetramethyl-4-piperidyl)-dimethylaminopropylamino]-1,3,5-triazin-6-yl}-tetraethylenpentamin
15. N,N',N'',N''',N$^{IV}$-Pentakis-{2,4-[N-(2,2,6,6-tetramethyl-4-piperidyl)-diethylaminopropylamino]-1,3,5-triazin-6-yl}-tetraethylenpentamin
16. N,N',N'',N''',N$^{IV}$,N$^{V}$-Hexakis-{2,4-[N-(2,2,6,6-tetramethyl-4-piperidyl)-dimethylaminopropylamino]-1,3,5-triazin-6-yl}-pentaethylenhexamin
17. N,N',N'',N''',N$^{IV}$,N$^{V}$-Hexakis-{2,4-[N-(2,2,6,6-tetramethyl-4-piperidyl)-diethylaminopropylamino]-1,3,5-triazin-6-yl}-pentaethylenhexamin

Die neuen Triazinstabilisatoren werden aus Cyanurhalogeniden erhalten, wobei man die Synthese nach zwei Varianten vollziehen kann. Nach Variante A wird zunächst ein substituiertes Triazin der Formel (III)

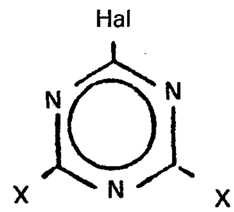

(III)

worin Hal = Halogen, bevorzugt Chlor, ist und X die oben angegebene Bedeutung hat, hergestellt, indem man 1 Mol eines Cyanurhalogenids mit 2 Mol eines Amins der Formel (VI)

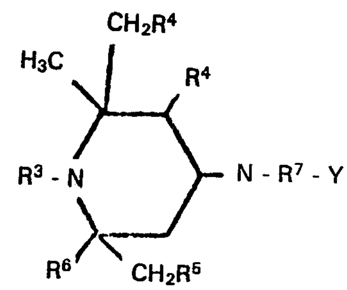

(VI)

in der $R^3$, $R^4$, $R^5$, $R^6$, $R^7$ und Y die oben angegebene Bedeutung haben und welches entsprechend Beispiel 17 der GB-A-834 290 zugänglich ist, umsetzt. Das hierbei erhaltene Produkt wird sodann mit der — bezogen auf Hal — äquivalenten Menge eines Polyamins der Formel (IV)

$$\underset{R^2}{\underset{|}{HN}}-R^1-[\underset{H}{\underset{|}{N}}(CH_2)_\ell]_m-\underset{H}{\underset{|}{N}}(CH_2)_n-\underset{R^2}{\underset{|}{NH}} \qquad (IV)$$

in der $R^1$, $R^2$, $\ell$, m und n die oben angegebenen Bedeutungen haben, zur Reaktion gebracht.

Nach Variante B lässt man zunächst ein Polyamin (IV) mit einem Cyanurhalogenid zu einer Verbindung der Formel (V)

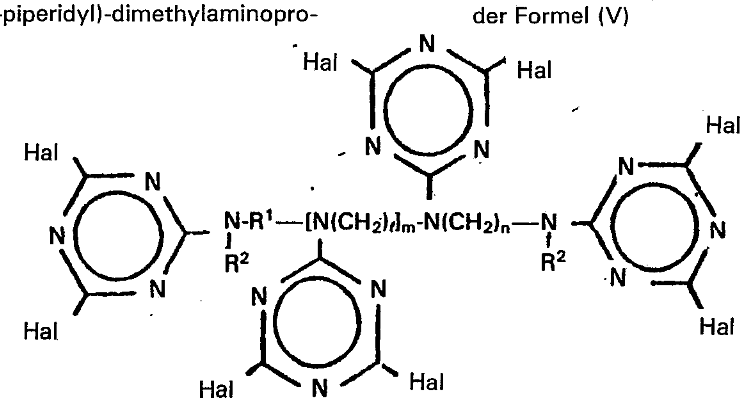

(V)

worin Hal = Halogen, bevorzugt jedoch Chlor, ist, und $R^1$, $R^2$, $\ell$, m sowie n die oben angegebenen Bedeutungen haben, abreagieren. Anschliessend wird dann mit der — bezogen auf Hal — äquivalenten Menge einer Verbindung der Formel (VI) umgesetzt.

Eine besonders einfache Arbeitsweise besteht darin, dass man die Zwischenverbindungen (III) bzw. (V), insbesondere jedoch (III), erst gar nicht isoliert, sondern nach deren Bildung sofort mit einem Polyamin der Formel (IV) bzw. einem Amin der Formel (VI) in einem sog. «Eintopfverfahren» zu den neuen Triazinstabilisatoren weiterreagieren lässt.

Amine der Formel (VI) sind beispielsweise:

(2,2,6,6-Tetramethyl-4-piperidyl)-3-dimethylaminopropylamin,

(2,2,6,6-Tetramethyl-4-piperidyl)-3-diethylaminopropylamin,

(2,2,6,6-Tetramethyl-4-piperidyl)-3-hydroxypropylamin,

(2,2,6,6-Tetramethyl-4-piperidyl)-3-ethoxypropylamin,

(2,2,6,6-Tetramethyl-4-piperidyl)-3-isooctoxypropylamin,

(2,2,6,6-Tetramethyl-4-piperidyl)-dimethylaminoethylamin und

(2,2,6,6-Tetramethyl-4-piperidyl)-4-diethylaminobutylamin.

Polyamine der Formel (IV) sind z.B.: Diethylentetramin, 1,8,15-Triaminopentadecan, 1,4,7,10-Tetraaminodecan, Tetraethylenpentamin, Pentaethylenhexamin.

Unter Cyanurhalogeniden werden bevorzugt die Chloride verstanden.

Die Reaktionen werden in organischen Lösungsmitteln, wie z.B. Petrolether, Aceton, Ether, Dioxan, Benzol, Toluol, Xylol, Cymol, Mesitylen usw. durchgeführt. Grundsätzlich sind zwei Reaktionsschritte bei unterschiedlichen Temperaturen erforderlich.

So werden die Zwischenverbindungen (III) bei 10 bis 40°C hergestellt und mit den Polyaminen (IV) bei 60 bis 150, vorzugsweise bei 80 bis 150°C zur Reaktion gebracht. Ist das Zwischenprodukt eine Verbindung der Formel (V), so arbeitet man zu dessen Herstellung zweckmässigerweise bei 0 bis 10°C und setzt dann mit dem Amin der Formel (VI) ebenfalls bei 60 bis 150, vorzugsweise bei 80 bis 150°C zu den gewünschten Endprodukten um.

Bei allen Reaktionsschritten ist es erforderlich, äquivalente Mengen, bezogen auf sich bildenden Halogenwasserstoff, einer Base, besonders eines Alkalimetallhydroxids in fester Form oder als wässrige Lösung, zuzusetzen. Geeignet sind z.B. Natriumhydroxid, Kaliumhydroxid, Natriumcarbonat und Kaliumcarbonat.

Es war überraschend und nicht vorhersehbar, dass die Herstellung der neuen Verbindungen in der erfindungsgemässen Weise möglich ist. Man musste vielmehr annehmen, dass die seit geraumer Zeit bekannte Umetzung von Cyanursäurehalogeniden mit primären oder sekundären Mono- oder Polyaminen, welche nach dem Prinzip der Carbonsäureamidsynthese aus Säurechloriden und Aminen vor sich geht [J. Am. Chem. Soc. 73 (1951), Nr. 7, S. 2981 ff; US-A-2 544 071; CH-A-342 784; CH-A-342 785], im vorliegenden Falle nicht störungsfrei ablaufen würde. Da die Schlüsselreaktion für die Herstellung der neuen Verbindungen in der Kondensation zwischen dem unsubstituierten oder substituierten Cyanurhalogenid und der Verbindung (VI)

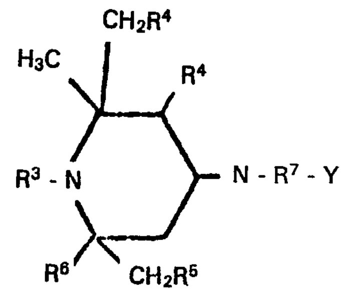

(VI)

besteht, diese jedoch eine zusätzliche funktionelle Gruppe Y, die $-OR^8$ und $-N(R^9)_2$ sein kann, besitzt, welche analog der -NH-Gruppe ebenfalls zu Reaktionen mit Säurechloriden befähigt ist, war nämlich damit zu rechnen, dass die Verbindungen (VI) als Brücken zwischen zwei verschiedenen Triazinringen fungieren, was zu unerwünschten vernetzten Produkten führen würde. Veröffentlichungen über die bekannte Bildung von Cyanursäureestern aus Alkoholen, welche während der Reaktion auch aus solchen Verbindungen mit $Y = -OR^8$ durch Etherspaltung aufgrund nicht auszuschliessender Mengen Halogenwasserstoff entstehen können, sowie über die Synthese von Cyanursäuredialkylaminen aus Cyanurhalogenid und tert. Aminen, wie z.B. Triethylamin, Dialkylarylamine, Alkylpiperidine und Alkylmorpholine [Ullmann Bd. 9 (1975), S. 651; E. Kober und R. Rätz, J. Org. Chem. 27 (1962), S. 2509 ff] liessen einen derartigen Reaktionsverlauf als durchaus möglich, wenn nicht sogar bevorzugt erscheinen.

Die neuen Triazinstabilisatoren lassen sich problemlos in die zu stabilisierenden Polymeren einarbeiten und sind hervorragend zum Stabilisieren derselben gegen den lichtinduzierten oxidativen Abbau geeignet.

Zum Stabilisieren von Polymeren sind Triazinverbindungen bereits vorgeschlagen worden (DE-A-2 636 144, DE-A-2 636 130 und SU-A-600 140), es hat sich jedoch gezeigt, dass diese noch mit diversen Mängeln behaftet sind. So ist es für die Beurteilung der Brauchbarkeit einer Substanz als Stabilisator von grosser Bedeutung, dass neben der Wirksamkeit einige weitere physikalische Eigenschaften vorhanden sind, insbesondere z.B. geringe Flüchtigkeit, Verträglichkeit mit den zu stabilisierenden Polymeren, Migrationsfestigkeit gegenüber Wasser, welche bei der Freibewitterung eine bedeutende Rolle spielt, und auch gegenüber Kohlenwasserstoffen, ein Schmelzpunkt, der unterhalb der für den Kunststoff notwendigen Verarbeitungstemperatur liegt und Voraussetzung für eine gleichmässige Verteilung desselben im Polymeren ist, sowie die Thermostabilität der Additive auch bei den z.T. sehr hohen Verarbeitungstemperaturen.

Es ist bisher noch keine Substanz bekannt, die in allen genannten Punkten diesen, an einen hervorragenden Stabilisator gestellten Anforderungen genügen würde, um im Praxiseinsatz voll befriedigen zu

können, und damit sind die in den obengenannten Druckschriften beschriebenen Triazinverbindungen bezüglich ihrer Stabilisatoreignung nur als Kompromisslösungen anzusehen. Aus diesen Gründen ist es auch verständlich, dass keines dieser Produkte bisher auf dem Markt Bedeutung erlangt hat. In grösserem Masse wird derzeit nur das Bis-(2,2,6,6-tetramethyl-4-piperidyl)-sebacat als Lichtschutzmittel eingesetzt (DE-C-1 929 928 und DE-A-2 204 659).

Die Triazinstabilisatoren der Erfindung sind weitgehend frei von den oben aufgezeigten Nachteilen und zeichnen sich durch eine ausserordentlich gute Stabilisatorwirksamkeit für organische Polymere aus. So sind sie trotz ihrer enormen Polarität, welche nicht zuletzt durch den Rest Y bedingt ist, unerwartet gut mit den zu stabilisierenden Polymeren verträglich, was besonders beim Einsatz in unpolaren Polymeren wie Polyethylen und Polypropylen als überraschend anzusehen ist. Auch die im Vergleich zu der besten Verbindung des Standes der Technik (Beispiel 6 der DE-A-2 636 144) geringe Flüchtigkeit war eben wegen des an sich weniger thermostabilen Y-Restes nicht vorhersehbar.

Die neuen Verbindungen werden wie bereits ausgeführt als Stabilisatoren für Kunststoffe gegen deren Schädigung durch Einwirkung von Sauerstoff, Wärme und Licht verwendet. Beispiele für solche Kunststoffe sind im einzelnen:

Polymere, die sich von einfach oder doppelt ungesättigten Kohlenwasserstoffen ableiten, z.B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrundeliegenden Monomeren, wie Ethylen-Propylen-Copolymere, Propylen-Buten-1-Copolymere, Propylen-Isobuten-Copolymere, Styrol-Butadien-Copolymere, sowie Terpolymere von Ethylen und Propylen mit einem Dien, wie z.B. Hexadien, Dicyclopentadien oder Ethylidennorbornen; Mischungen der obengenannten Homopolymeren, wie beispielsweise Gemische von Polypropylen und Polyethylen, Polypropylen und Polybuten-1, Polypropylen und Polyisobutylen oder von Butadien-Acrylnitril-Copolymerisat mit einem Styrol-Butadien-Copolymerisat.

Halogenhaltige Vinylpolymere, wie Polyvinylchlorid, Polyvinylidenchlorid, Polyvinylfluorid, Polychloropren und Chlorkautschuke, sowie Copolymere von Vinylchlorid und Vinylidenchlorid untereinander und mit anderen olefinisch ungesättigten Monomeren.

Polymere, die sich von $\alpha,\beta$-ungesättigten Säuren und deren Derivaten ableiten, wie Polyacrylate und Polymethacrylate, Polyacrylamide und Polyacrylnitril, sowie deren Copolymere untereinander und mit anderen Vinylverbindungen, wie Acrylnitril-Butadien-Styrol-, Acrylnitril-Styrol- und Acrylnitril-Styrol-Acrylester-Copolymerisate.

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen-Vinylacetat-Copolymere.

Homo- und Copolymere, die sich von Epoxiden ableiten, wie Polyethylenoxid oder die Polyymerisate, die sich von Bisglycidylethern ableiten.

Polyacetale, wie Polyoxymethylen und Polyethylen, sowie solche Polyoxymethylene, die als Comonomeres Ethylenoxid enthalten.

Polyurethane und Polyharnstoffe.

Polycarbonat.

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylol-cyclohexanterephthalat.

Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

Von besonderer Bedeutung ist die Stabilisierung von Polyolefinen, Styrolpolymerisaten, Polyamiden, Poly(meth-)acrylaten und von Polyurethanen, für die sich die Verbindungen bevorzugt eignen. Beispiele hierfür sind Polyethylen hoher und niedriger Dichte, Polypropylen, Ethylen-Propylen-Copolymerisate, Polystyrol, Styrol-Butadien-Acrylnitril-Terpolymerisate, Mischungen von Polyolefinen oder von Styrolpolymerisaten sowie Polyurethane auf Polyether- oder Polyesterbasis.

Die neuen Stabilsatoren werden nach allgemein üblichen Methoden in die Polymermassen eingearbeitet. Die Einarbeitung kann beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere direkt oder Einmischen in eine Lösung, Suspension oder Emulsion desselben, gegebenenfalls unter nachträglichem Verdunstenlassen des Lösungsmittels, erfolgen. Die Mengen liegen bei 0,01 bis 5, vorzugsweise bei 0,05 bis 2,5 und insbesondere bei 0,1 bis 1,0 Gew.-%, bezogen auf das zu stabilisierende Material. Die neuen Verbindungen können auch in Form eines Masterbatches, der diese Verbindungen beispielsweise in einer Konzentration von 2,5 bis 50, vorzugsweise 5,0 bis 20 Gew.-% enthält, den zu stabilisierenden Kunststoffen zugesetzt werden.

Die durch den Zusatz der erfindungsgemässen Substanzen stabilisierten Kunststoffe können gegebenenfalls noch andere bekannte und übliche Zusätze, wie beispielsweise Antioxidantien auf Phenol- und Sulfidbasis, UV-Absorber und Lichtschutzmittel, Phosphitstabilisatoren, Metallverbindungen, Epoxystabilisatoren und mehrwertige Alkohole enthalten.

Beispiele für Antioxidantien sind solche vom Typ der sterisch gehinderten Phenole wie 4,4'-Butyliden-bis-(2,6-di-tert.-butylphenol), 4,4'-Thio-bis-(2-tert.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z.B. 2-(2'-Hydroxyphenyl)-benztriazole wie 2-(2'-Hydroxy-5'-methylphenyl)-benztriazol, 2-Hydroxybenzophenone wie 2-Hydroxy-4-octoxy-benzophenon, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Barium-, Strontium-, Zink-, Cadmium-, Magnesium-, Aluminium- und Bleiseifen aliphatischer Carbonsäuren oder Oxycarbonsäuren mit 12 bis 32 C-Atomen, Salze der genannten Metalle mit aromatischen Carbonsäuren wie Benzoate oder Salizylate sowie (Alkyl-)Phenolate dieser Metalle, ferner Organozinnverbindungen, wie z.B. Dialkylzinnthioglykolate und -carboxylate.

Bekannte Epoxystabilsatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d.h. bevorzugt Alkohole mit 5 oder 6 C-Atomen und 2 bis 6 OH-Gruppen.

Eine wirksame Stabilsatorkombination für Poly-$\alpha$-Olefine, wie z.B. Hoch-, Mittel- und Niederdruckpolymerisate von $C_2$- bis $C_4$-$\alpha$-Olefinen, insbesondere Polyethylen und Polypropylen oder von Copolymerisaten derartiger $\alpha$-Olefine besteht, bezogen auf 100 Gewichtsteile Polymer, beispielsweise aus 0,01 bis 5 Gewichtsteilen eine der erfindungsgemäss zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, 4-Hydroxyphenylbenzotriazole, Benzylidenmalonsäuremononitrilester oder der sog. Quencher, wie z.B. Nickelchelate.

Die erfindungsgemäss stabilisierten Kunststoffe können in verschiedenster Form angewendet werden, z.B. als Folien, Fasern, Bändchen, Profile oder als Bindemittel für Lacke, Klebemittel oder Kitte.

*Anwendungstechnische Untersuchungen*

a) *Flüchtigkeit*

Die Flüchtigkeiten werden in einer Apparatur zur thermogravimetrischen Analyse bestimmt. Gleiche Mengen (500 mg) der erfindungsgemässen Verbindungen und der Vergleichssubstanz werden dazu in Stickstoffatmosphäre mit einer Aufheizgeschwindigkeit von 2K/min bis auf 300°C erhitzt und der Substanzverlust in mg/cm$^2$ gemessen.

b) *Lichtstabilisierende Wirkung der neuen Verbindungen in Poly-$\alpha$*-Olefinen

1) 100 Gewichtsteile Polypropylen mit einem Schmelzindex $i_5$ von ca 6 g/10 min (bestimmt nach ASTM D 1238-62 T) und einer Dichte von 0,96 g/cm$^3$ werden mit

| | |
|---|---|
| 0,1 Gew.-Teilen | Pentaerythrityl-tetrakis-3-(3,5-di--tert.-butyl-4-hydroxyphenyl)--propionat, |
| 0,2 Gew.-Teilen | Calciumstearat und |
| 0,1 Gew.-Teilen | des zu prüfenden erfindungsgemässen Stabilisators vermischt. |

Um eine möglichst gleichmässige Verteilung auf dem Polymerkorn zu erreichen, werden die Stabilisatoren in einem Lösemittel gelöst, und die Lösung wird unter Rühren in das Polypropylenpulver eingetropft, wobei durch gleichzeitige Bestrahlung mit einer IR-Lampe der grösste Teil des Lösemittels wieder abdampft.

Nach ca. 20 min wird das Calciumstearat hinzugegeben und noch weiter 10 min gemischt. Lösemittelreste wurden durch Trocknen bei 50°C/120 min im Trockenschrank entfernt.

Das Polypropylen wird auf einer Windsor-Spritzgussmaschine der Type SP 50 bei 240°C zu 60 × 60 × 1 mm-Platten verspritzt. Aus diesen Platten werden Prüfkörper nach DIN 53 455, Form 3, verkleinert im Massstab 1 : 3, ausgestanzt. Die als Vergleichsmuster benötigten Prüfkörper werden analog, jedoch unter Fortlassen des zu testenden Stabilisators bzw. unter Zusatz der Vergleichsstabilisatoren, hergestellt.

Zur Bestimmung der Lichtstabilität werden die Proben in einer Xenotest-1200-Apparatur der Firma Original Hanau Quarzlampen GmbH der Bestrahlung mit Wechsellicht unterworfen. Die Strahlungsintensität wird durch UV-Filter (Spezialfilterglas d = 1,7 mm) moduliert. Die Lichtbeständigkeit wird nach DIN 53 387 (17 min befeuchten, 3 min beregnen, Schwarztafeltemperatur 45°C, Luftfeuchtigkeit 70 bis 75%) geprüft. Gemessen wird die Belichtungszeit in Stunden und die Reissdehnung auf einer Zugprüfmaschine der Firma Instron bei einer Abzugsgeschwindigkeit von 5 cm/min ermittelt.

2) Zu Polypropylen (® Hostalen PPU VP 1770 F der HOECHST AG) vom Schmelzindex MFI 190/5 : 19 g/10 min s. DIN 53 535 werden 0,1 bis 0,25 Gew.-Teile der Stabilisatoren über einen Laborschnellmischer eingemischt. Das so stabilisierte Material wird dann in einem Laborextruder unter den üblichen Verarbeitungsbedingungen aufgeschmolzen und über eine Spinnpumpe mit Mehrfachspinnkopf zu Monofilamenten (87 dtex) verarbeitet, welche anschliessend im Verhältnis 1 : 2,5 nachverstreckt wurden. Je 24 dieser Filamente werden zu Garn texturiert und diese zu Prüfgeweben verarbeitet. Die Prüflinge werden in einem Fadeometer der Lichtechtheitsprüfung unterzogen und nach der angegebenen Belichtungszeit dem Fingernageltest (leichtes Darüberreiben über das Gewebe mit dem Daumennagel) unterzogen. Nach 160 Stunden Belichtungszeit zeigten die mit den erfindungsgemässen Verbindungen stabilisierten Prüfgewebe noch keine Schädigung.

Die folgenden Beispiele dienen der weiteren Erläuterung der Erfindung. Beispiele 1 bis 4 zeigen die Herstellung von Zwischenverbindungen der Formel (III), welche z.B. nach Beispiel 5 weiter umgesetzt werden.

*Beispiel 1*

*2,4-[N-(2,2,6,6-Tetramethyl-4-piperidyl)-3--diethylaminopropylamino]-6-chlor-1,3,5-triazin [Zwischenverbindung der Formel (III)]*

Zu einer Lösung von 269 g (1 Mol) N,N-Diethyl-N'--(2,2,6,6-tetramethyl-4-piperidinyl)-propan-1,3-diamin in 500 ml Aceton wird bei 20°C eine Lösung aus 92,2 g (0,5 Mol) Cyanurchlorid in 500 ml Aceton getropft. Nach 30minütigem Rühren wird bei 20°C eine Lösung aus 40 g (1 Mol) NaOH und 100 ml Wasser zugefügt und 6 Stunden bei 20°C nachgerührt. Das ausgefallene NaCl wird abfiltriert und das Filtrat bei Raumtemperatur einrotiert. Der Rückstand wird über $CaCl_2$ getrocknet.

*Beispiel 2*

*2,4-[N-(2,2,6,6-Tetramethyl-4-piperidyl)-4--diethylaminobutylamino]-6-chlor-1,3,5-triazin [Zwischenverbindung der Formel (III)]*

Diese Substanz wird analog Beispiel 1 aus 283 g (1 Mol) N,N-Diethyl-N'-(2,2,6,6-tetramethyl-4-piperidinyl)-butan-1,4-diamin und 92,2 g (0,5 Mol) Cyanurchlorid hergestellt.

*Beispiel 3*

*2,4-[N-(2,2,6,6-Tetramethyl-4-piperidyl)-3--ethoxypropylamino]-6-chlor-1,3,5-triazin [Zwischenverbindung der Formel (III)]*

48,4 g (0,2 Mol) N-(2,2,6,6-Tetramethyl-4-piperidinyl)-3-ethoxy-propan-1-amin werden in 100 ml Aceton vorgelegt. Dazu wird bei 20°C eine Lösung aus 18,5 g (0,1 Mol) Cyanurchlorid und 250 ml Aceton getropft. Es wird 6 Stunden bei 40°C gerührt und sodann das Lösungsmittel im Vakuum abrotiert. Der Rückstand wird in Toluol aufgenommen, filtriert und das Filtrat einrotiert. Es bleibt ein festes Harz zurück.

Cl: gef. 6,9% ber. 6,3%

*Beispiel 4*

*2,4-[N-(2,2,6,6-Tetramethyl-4-piperidyl)-3--dimethylaminopropylamino]-6-chlor-1,3,5-triazin [Zwischenverbindung der Formel (III)]*

Diese Verbindung wurde analog Beispiel 1 aus 241 g (1 Mol) N,N-Dimethyl-N'-(2,2,6,6-tetramethyl)-4-piperidinyl-propan-1,3-diamin und 92,2 g (0,5 Mol) Cyanurchlorid hergestellt.

*Beispiel 5*

*N,N',N'',N''',N^IV-Pentakis-{2,4-[N-(2,2,6,6-tetramethyl-4-piperidyl)-diethylaminopropylamino]--1,3,5-triazin-6-yl}-tetraethylenpentamin*

In 100 ml Xylol werden 32,5 g (0,05 Mol) des Produktes nach Beispiel 1, 1,83 g (0,01 Mol) Tetraethylenpentamin und 1,6 g (0,04 Mol) NaOH-Pulver vorgelegt. Man kocht das Gemisch 15 Stunden am Wasserabscheider, wobei ca. 0,5 ml Wasser abgetrennt werden. Anschliessend wird das NaCl abfiltriert, das Filtrat einrotiert und der Rückstand bei 180°C im Hochvakuum getrocknet. Es blieb ein harziges festes Produkt.

*Beispiele 6 bis 11*

Beispiel 6 zeigt die Arbeitsweise nach dem «Eintopfverfahren».

*Beispiel 6*

*N,N'N''-Tris-{2,4-[N-(2,2,6,6-tetramethyl-4--piperidyl)-3-diethylaminopropylamino]-1,3,5--triazin-6-yl}-diethylendiamin*

Es werden 26,9 g (0,1 Mol) N,N-Diethyl-N'--(2,2,6,6-tetramethyl-4-piperidyl)-propan-1,3-diamin (Monoamin) in 100 ml Toluol vorgelegt. Dazu wird bei 20 bis 30°C eine Lösung aus 100 ml Toluol und 9,2 g (0,05 Mol) Cyanurchlorid getropft. Anschliessend fügt man 4,0 g (0,1 Mol) NaOH-Pulver hinzu und rührt 6 Stunden bei 30°C nach. Man gibt sodann 1,7 g (0,017 Mol) Diethylentriamin (Polyamin) und 2,0 g (0,05 Mol) NaOH-Pulver zu, kocht weitere 16 Stunden am Wasserabscheider, filtriert hierauf heiss das ausgeschiedene NaCl ab und entfernt das Lösungsmittel im Vakuum bei zuletzt 150°C. Es bleibt eine helle feste Substanz vom Fp. 146°C zurück.

*Beispiele 7 bis 11*

Es wird analog Beispiel 6 unter Einsatz des gleichen Monoamins gearbeitet.

| Beispiel Nr. | Polyamin / g | Fp (°C) |
|---|---|---|
| 7 | Dipropylentriamin / 2,2 | 109 |
| 8 | 3-(2-Aminoethyl)-aminopropylamin / 1,9 | 97 |
| 9 | Triethylentetramin / 1,8 | 119 |
| 10 | 4,7-Diazadecan-1,10-diamin / 2,2 | 120 |
| 11 | Pentaethylenhexamin / 1,9 | 90 |

*Beispiel 12*

*N,N',N'',N'''-Tetrakis-{2,4-[N-(2,2,6,6-tetramethyl-4-piperidyl)-3-dimethylaminopropylamino]--1,3,5-triazin-6-yl}-triethylentetramin*

Die Herstellung erfolgt analog Beispiel 6 unter Einsatz von 24,1 g N,N-Dimethyl-N'-(2,2,6,6-tetramethyl-4-piperidyl)-propan-1,3-diamin und 1,8 g Triethylentetramin. Man erhält ein Produkt vom Fp. 73 bis 87°C.

**Patentansprüche für die Vertragsstaaten:**
BE, CH, DE, FR, GB, IT, LI, NL, SE

1. Triazinverbindungen der Formel (I)

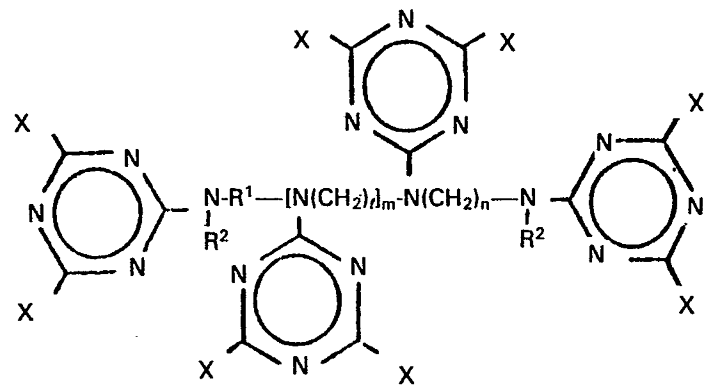

(I)

in der
$R^2$ für Wasserstoff, eine $C_1$- bis $C_6$-Alkylgruppe oder eine Gruppe der Formel (II)

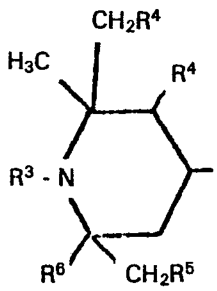

(II)

steht, in welcher
$R^3$ Wasserstoff oder $C_1$- bis $C_{18}$-Alkyl, ist
$R^4$ und $R^5$ entweder gleich sind und Wasserstoff oder eine $C_1$- bis $C_5$-Alkylgruppe bedeuten, wobei dann
$R^6$ eine Methylgruppe ist, oder
$R^4$ Wasserstoff oder $C_1$- bis $C_5$-Alkyl ist und
$R^5$ und $R^6$ zusammen mit den Kohlenstoffatomen, an die sie gebunden sind, einen $C_5$- oder $C_6$-Cycloalkylring oder eine Gruppe der Formel

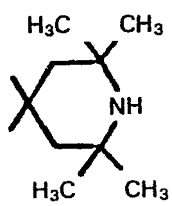

darstellen,
X die Bedeutung einer Gruppe der Formel (III)

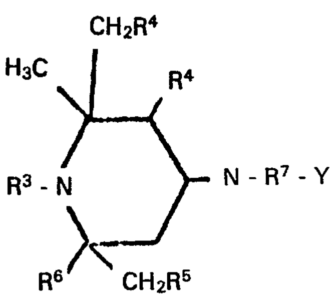

(III)

hat, in welcher $R^3$, $R^4$, $R^5$ und $R^6$ die bereits angegebene Bedeutung haben,
$R^7$ für eine Alkylengruppe mit 2 bis 4 C-Atomen, die durch eine Methylgruppe substituiert sein kann, und
Y für eine Gruppe der Formeln $-OR^8$ oder $-N(R^9)_2$ steht, mit $R^8$ = $C_1$- bis $C_{18}$-Alkyl und $R^9$ = Methyl oder Ethyl, und
$R^1$ wenn m eine ganze Zahl von 0 bis 3 ist, eine Alkylengruppe der Formel $-(CH_2)_r-$ bedeutet, wobei im Falle von m = 0 die Indices r und n gleich oder verschieden sein können und für eine ganze Zahl von 2 bis 6 stehen , und im Falle von m = 1, 2 oder 3 jedoch der Index $\ell$ für 2 oder 3 steht und die Indices r sowie n gleich sind und ebenfalls die Bedeutung von 2 oder 3 haben.

2. Verfahren zur Herstellung der Verbindungen nach Anspruch 1, dadurch gekennzeichnet, dass man entweder

A) aus 1 Mol Cyanurhalogenid und 2 Mol eines Amins der Formel (VI)

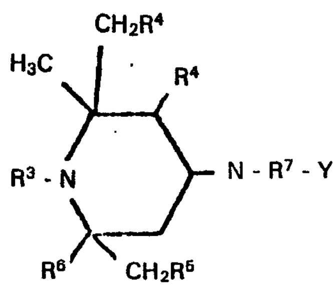

(VI)

in der $R^3$, $R^4$, $R^5$, $R^6$, $R^7$ und Y die in Anspruch 1 angegebene Bedeutung haben, in Anwesenheit von 2 Mol einer Base und einem organischen Lösungsmittel bei 10 bis 40°C eine Verbindung der Formel (III)

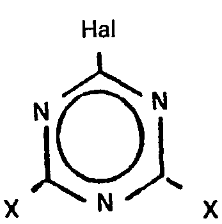

(III)

worin Hal = Halogen und X der Rest des Amins der Formel (VI) ist, synthetisiert, worauf diese in einem inerten organischen Lösungsmittel bei einer Reaktionstemperatur von 60 bis 150°C mit der dem Hal äquivalenten Menge eines Polyamins der Formel (IV)

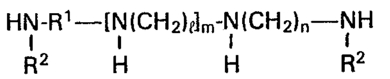

(IV)

in der $R^1$, $R^2$, $\ell$, m und n die in Anspruch 1 angegebene Bedeutung haben, in Gegenwart einer — bezogen auf sich bildenden Halogenwasserstoff — äquivalenten Menge einer Base umgesetzt wird, oder

B) ein Polyamin (IV) in einem inerten organischen Lösungsmittel bei 0 bis 10°C mit der äquimolaren Menge — bezogen auf Aminofunktionen — eines Cyanurhalogenides in Anwesenheit äquimolarer Mengen einer Base — bezogen auf sich bildenden Halogenwasserstoff — zu einer Verbindung der Formel (V)

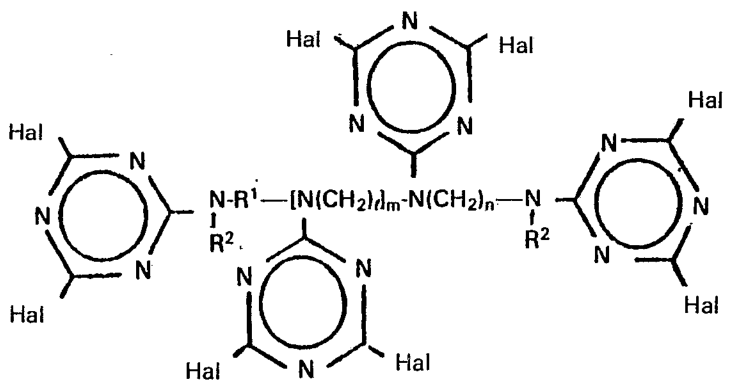

(V)

derivatisiert wird, worauf man diese bei 60 bis 150°C in einem inerten organischen Lösungsmittel mit der — bezogen auf Halogenreste — äquivalenten Menge einer Verbindung der oben angegebenen Formel (VI) in Gegenwart der — bezogen auf entstehenden Halogenwasserstoff — äquimolaren Menge einer Base zur Reaktion bringt.

3. Verfahren nach Anspruch 2, dadurch gekennzeichnet, dass die Zwischenverbindungen (III) bzw. (V) nicht isoliert werden.

4. Verwendung der Verbindungen nach Anspruch 1 zum Stabilisieren von synthetischen Polymeren.

5. Verwendung nach Anspruch 4, dadurch gekennzeichnet, dass die Polymeren Polyolefine, halogenhaltige Polymere, Polyacrylate oder Polymethacrylate oder Homo- oder Copolymere von Styrol sind.

6. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

**Patentansprüche für den Vertragsstaat:** AT

1. Verfahren zur Herstellung von Triazinverbindungen der Formel (I)

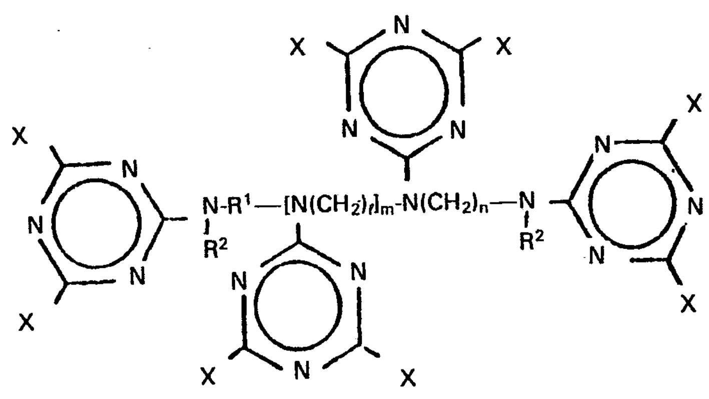

(I)

in der

$R^2$ für Wasserstoff, eine $C_1$- bis $C_6$-Alkylgruppe oder eine Gruppe der Formel (II)

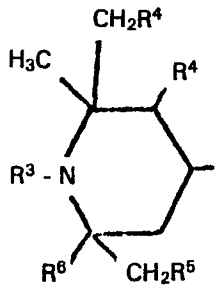
(II)

steht, in welcher

$R^3$ Wasserstoff oder $C_1$- bis $C_{18}$-Alkyl, ist

$R^4$ und $R^5$ entweder gleich sind und Wasserstoff oder eine $C_1$- bis $C_5$-Alkylgruppe bedeuten, wobei dann

$R^6$ eine Methylgruppe ist, oder

$R^4$ Wasserstoff oder $C_1$- bis $C_5$-Alkyl ist und

$R^5$ und $R^6$ zusammen mit den Kohlenstoffatomen, an die sie gebunden sind, einen $C_5$- oder $C_6$-Cycloalkylring oder eine Gruppe der Formel

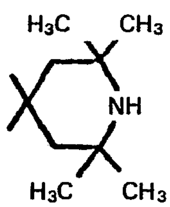

darstellen,

X die Bedeutung einer Gruppe der Formel (III)

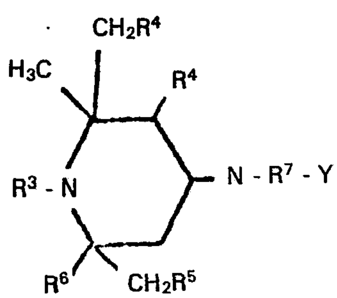
(III)

hat, in welcher $R^3$, $R^4$, $R^5$ und $R^6$ die bereits angegebene Bedeutung haben,

$R^7$ für eine Alkylengruppe mit 2 bis 4 C-Atomen, die durch eine Methylgruppe substituiert sein kann, und

Y für eine Gruppe der Formeln $-OR^8$ oder $-N(R^9)_2$ steht, mit $R^8$ = $C_1$- bis $C_{18}$-Alkyl und $R^9$ = Methyl oder Ethyl, und

$R^1$ wenn m eine ganze Zahl von 0 bis 3 ist, eine Alkylengruppe der Formel $-(CH_2)_r-$ bedeutet, wobei im Falle von m = 0 die Indices r und n gleich oder verschieden sein können und für eine ganze Zahl von 2 bis 6, und im Falle von m = 1, 2 oder 3 jedoch der Index $\ell$ für 2 oder 3 steht und die Indices r sowie n gleich sind und ebenfalls für 2 oder 3 stehen, dadurch gekennzeichnet, dass man entweder

A) aus 1 Mol Cyanurhalogenid und 2 Mol eines Amins der Formel (VI)

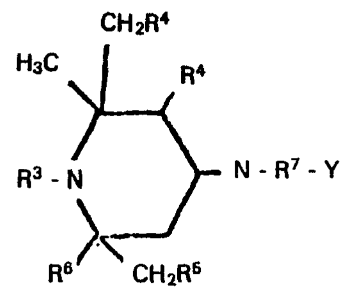
(VI)

in der $R^3$, $R^4$, $R^5$, $R^6$, $R^7$ und Y die in Anspruch 1 angegebene Bedeutung haben, in Anwesenheit von 2 Mol einer Base und einem organischen Lösungsmittel bei 10 bis 40°C eine Verbindung der Formel (III)

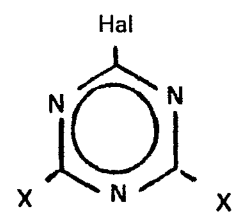
(III)

worin Hal = Halogen und X der Rest des Amins der Formel (VI) ist, synthetisiert, worauf diese in einem inerten organischen Lösungsmittel bei einer Reaktionstemperatur von 60 bis 150°C mit der dem Hal äquivalenten Menge eines Polyamins der Formel (IV)

$$\underset{R^2}{HN}-R^1-[\underset{H}{N}(CH_2)_\ell]_m-\underset{H}{N}(CH_2)_n-\underset{R^2}{NH} \qquad (IV)$$

in der $R^1$, $R^2$, $\ell$, m und n die in Anspruch 1 angegebene Bedeutung haben, in Gegenwart einer — bezogen auf sich bildenden Halogenwasserstoff — äquivalenten Menge einer Base umgesetzt wird, oder

B) ein Polyamin (IV) in einem inerten organischen Lösungsmittel bei 0 bis 10°C mit der äquimolaren Menge — bezogen auf Aminofunktionen — eines Cyanurhalogenides in Anwesenheit äquimolarer Mengen einer Base — bezogen auf sich bildenden Halogenwasserstoff — zu einer Verbindung der Formel (V)

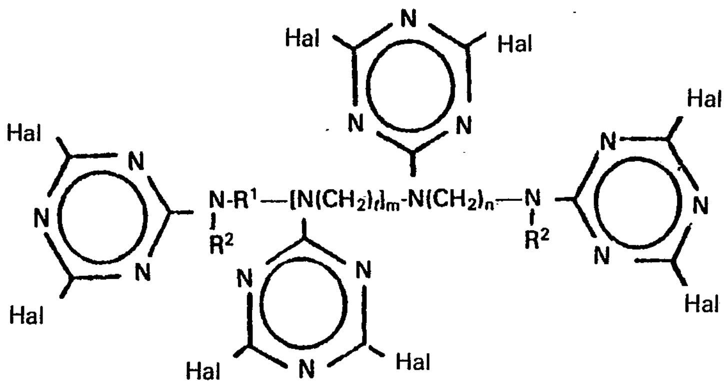

(V)

derivatisiert wird, worauf man diese bei 60 bis 150°C in einem inerten organischen Lösungsmittel mit der — bezogen auf Halogenreste — äquivalenten Menge einer Verbindung der oben angegebenen Formel (VI) in Gegenwart der — bezogen auf entstehenden Halogenwasserstoff — äquimolaren Menge einer Base zur Reaktion bringt.

2. Verfahren nach Anspruch 1, dadurch gekennzeichnet, dass die Zwischenverbindungen (III) bzw. (V) nicht isoliert werden.

3. Verwendung der nach Anspruch 1 und 2 hergestellten Verbindungen zum Stabilisieren von synthetischen Polymeren.

4. Verwendung nach Anspruch 3, dadurch gekennzeichnet, dass die Polymeren Polyolefine, halogenhaltige Polymere, Polyacrylate oder Polymethacrylate oder Homo- oder Copolymere von Styrol sind.

5. Verfahren zum Stabilisieren von synthetischen Polymeren gegen den schädigenden Einfluss von Licht, dadurch gekennzeichnet, dass man den Polymeren, gegebenenfalls neben bisher bekannten, stabilisierend wirkenden Stoffen, 0,01 bis 5 Gewichtsteile, bezogen auf Polymeres, eines nach Anspruch 1 und 2 hergestellten Stabilisators zusetzt.

**Claims for the Contracting States:**
BE, CH, DE, FR, GB, IT, LI, NL, SE

1. Triazine compounds of the formula (I)

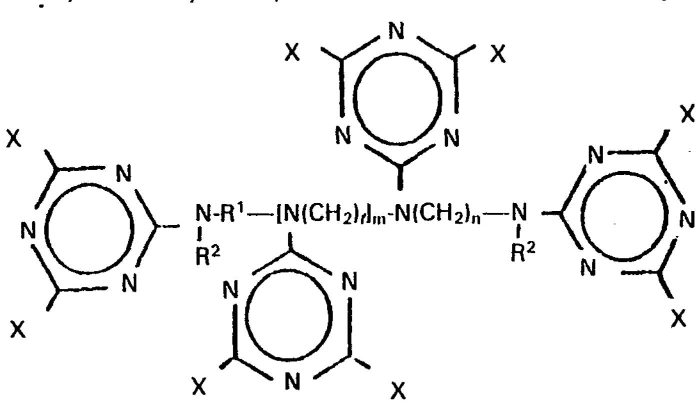

(I)

in which $R^2$ represents hydrogen, a $C_1$- to $C_6$-alkyl group, or a group of the formula (II)

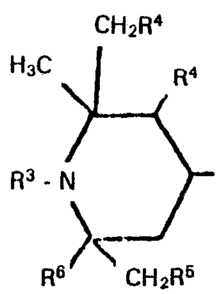

(II)

in which $R^3$ is hydrogen or $C_1$- to $C_{18}$-alkyl, $R^4$ and $R^5$ are either identical and denote hydrogen or a $C_1$- to $C_5$-alkyl group, in which case $R^6$ is a methyl group, or $R^4$ is hydrogen or $C_1$- to $C_5$-alkyl, and $R^5$ and $R^6$, together with the carbon atoms to which they are bonded, represent a $C_5$- or $C_6$-cycloalkyl ring or a group of the formula

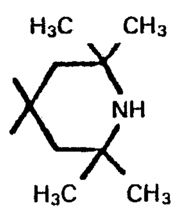

X denotes a group of the formula (III)

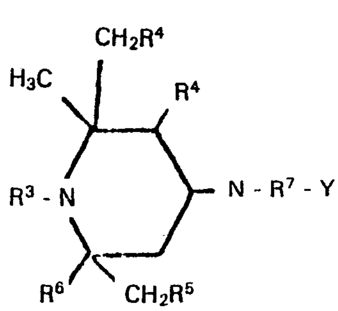

(III)

in which $R^3$, $R^4$, $R^5$ and $R^6$ have the meaning already given, $R^7$ represents an alkylene group which has 2 to 4 C atoms and can be substituted by a methyl group and Y represents a group of the formula $-OR^8$ or $-N(R^9)_2$, in which $R^8$ = $C_1$- to $C_{18}$-alkyl and $R^9$ = methyl or ethyl, and, if m is an integer from 0 to 3, $R^1$ denotes an alkylene group of the formula $-(CH_2)_r-$, in which, in the case where m = 0, the indices r and n can be identical or different and represent an integer from 2 to 6, but in the case where m = 1, 2 or 3, the index $\ell$ represents 2 or 3 and the indices r and n are identical and likewise represent 2 or 3.

2. A process for the preparation of the compounds as claimed in claim 1, which comprises either A. synthesizing, from 1 mole of cyanuric halide and 2 moles of an amine of the formula (VI)

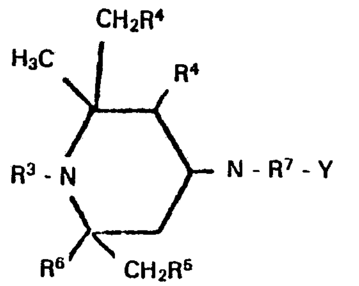

(VI)

in which $R^3$, $R^4$, $R^5$, $R^6$, $R^7$ and Y have the meaning given in claim 1, a compound of the formula (III)

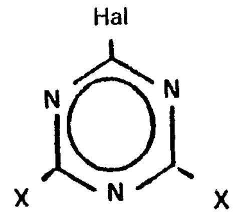

(III)

wherein Hal = halogen and X is the radical of the amine of the formula (VI), in the presence of 2 moles of a base and an organic solvent at 10 to 40°C, after which this product is reacted with the polyamine of the formula (IV)

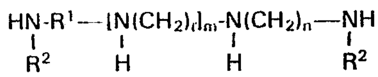

(IV)

in which $R^1$, $R^2$, $\ell$, m and n have the meaning given in claim 1, in an amount equivalent to the halogen, in an inert organic solvent at a reaction temperature of 60 to 150°C in the presence of an equivalent amount, relative to the hydrogen halide formed, of a base, or B. forming a derivative of a polyamine (IV) in an inert organic solvent at 0 to 10°C with an equimolar amount, relative to the amino functions, of a cyanuric halide in the presence of equimolar amounts of a base, relative to the hydrogen halide formed, to form a compound of the formula (V)

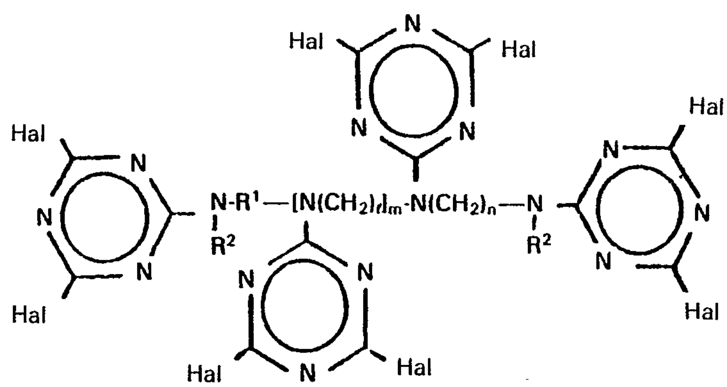

(V)

after which this product is reacted with the equivalent amount, relative to the halogen radicals, of a compound of the abovementioned formula (VI) at 60 to 150°C in an inert organic solvent in the presence of the equimolar amount, relative to the hydrogen halide formed, of a base.

3. A process as claimed in claim 2, wherein the intermediate compounds (III) or (V) are not isolated.

4. Use of the compounds as claimed in claim 1 for stabilizing synthetic polymers.

5. Use as claimed in claim 4, wherein the polymers are polyolefins, halogen-containing polymers, polyacrylates or polymethacrylates and homopolymers or copolymers of styrene.

6. A process for stabilizing synthetic polymers against the harmful effect of light, which comprises adding to the polymers 0.01 to 5 parts by weight, relative to the polymer, of a stabilizer as claimed in claim 1, if appropriate in addition to substances which have a stabilizing action and were hitherto known.

**Claims for the Contracting State:**
AT

1. A process for the preparation of triazine compounds of the formula (I)

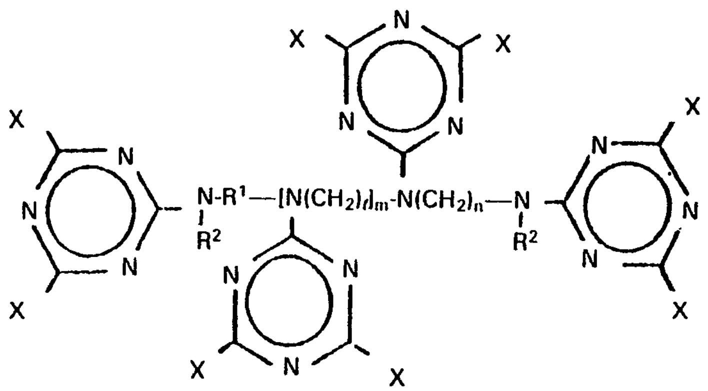

(I)

in which $R^2$ represents hydrogen, a $C_1$- to $C_6$-alkyl group, or a group of the formula (II)

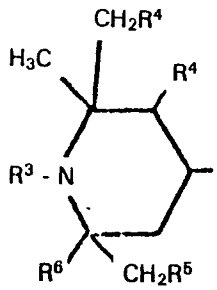

(II)

in which $R^3$ is hydrogen or $C_1$- to $C_{18}$-alkyl, $R^4$ and $R^5$ are either identical and denote hydrogen or a $C_1$- to $C_5$-alkyl group, in which case $R^6$ is a methyl group, or $R^4$ is hydrogen or $C_1$- to $C_5$-alkyl, and $R^5$ and $R^6$, together with the carbon atoms to which they are bonded, represent a $C_5$- or $C_6$-cycloalkyl ring or a group of the formula

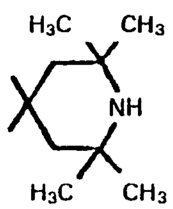

X denotes a group of the formula (III)

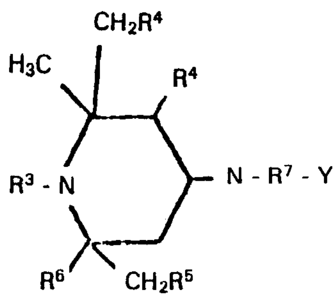

(III)

in which $R^3$, $R^4$, $R^5$ and $R^6$ have the meaning already given, $R^7$ represents an alkylene group which has 2 to 4 C atoms and can be substituted by a methyl group and Y represents a group of the formula $-OR^8$ or $-N(R^9)_2$, in which $R^8$ = $C_1$- to $C_{18}$-alkyl and $R^9$ = methyl or ethyl, and, if m is an integer from 0 to 3, $R^1$ denotes an alkylene group of the formula $-(CH_2)_r-$, in which, in the case where m = 0, the indices r and n can be identical or different and represent an integer from 2 to 6, but in the case where m = 1, 2 or 3, the index ℓ represents 2 or 3 and the indices r and n are identical and likewise represent 2 or 3, which comprises either

A. synthesizing, from 1 mole of cyanuric halide and 2 moles of an amine of the formula (VI)

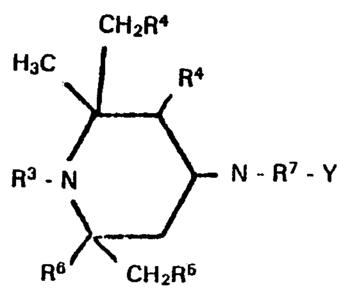

(VI)

in which $R^3$, $R^4$, $R^5$, $R^6$, $R^7$ and Y have the meaning given in claim 1, a compound of the formula (III)

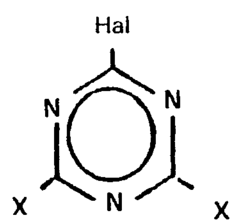

(III)

wherein Hal = halogen and X is the radical of the amine of the formula (VI), in the presence of 2 moles of a base and an organic solvent at 10 to 40°C, after which this product is reacted with the polyamine of the formula (IV)

$$HN(R^2)-R^1-[N(H)(CH_2)_\ell]_m-N(H)(CH_2)_n-NH(R^2) \qquad (IV)$$

in which $R^1$, $R^2$, ℓ, m and n have the meaning given in claim 1, in an amount equivalent to the halogen, in an inert organic solvent at a reaction temperature of 60 to 150°C in the presence of an equivalent amount, relative to the hydrogen halide formed, of a base, or

B. forming a derivative of a polyamine (IV) in an inert organic solvent at 0 to 10°C with an equimolar amount, relative to the amino functions, of a cyanuric halide in the presence of equimolar amounts of a base, relative to the hydrogen halide formed, to form a compound of the formula (V)

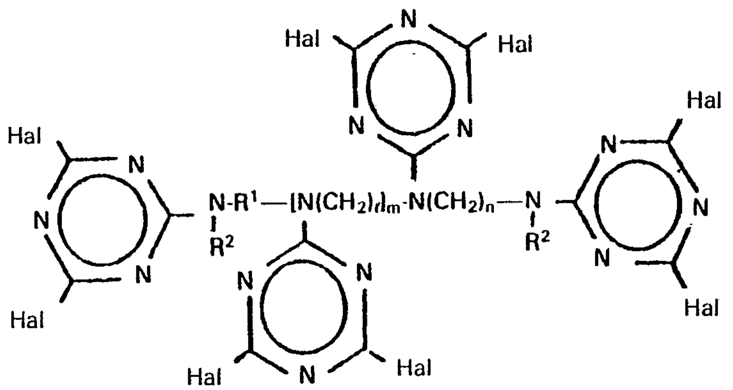

(V)

after which this product is reacted with the equivalent amount, relative to the halogen radicals, of a compound of the abovementioned formula (VI) at 60 to 150°C in an inert organic solvent in the presence of the equimolar amount, relative to the hydrogen halide formed, of a base.

2. A process as claimed in claim 1, wherein the intermediate compounds (III) or (V) are not isolated.

3. Use of the compounds as claimed in claim 1 and 2 for stabilizing synthetic polymers.

4. Use as claimed in claim 3, wherein the polymers are polyolefins, halogen-containing polymers, polyacrylates or polymethacrylates and homopolymers or copolymers of styrene.

5. A process for stabilizing synthetic polymers against the harmful effect of light, which comprises adding to the polymers 0.01 to 5 parts by weight, relative to the polymer, of a stabilizer as claimed in claim 1 and 2, if appropriate in addition to substances which have a stabilizing action and were hitherto known.

**Revendications pour les Etats Contractants:**
BE, CH, DE, FR, GB, IT, LI, NL, SE

1. Composés triaziniques répondant à la formule (I):

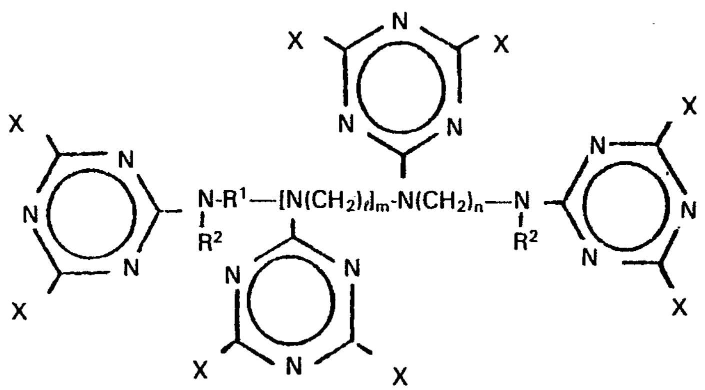

(I)

dans laquelle
$R^2$ désigne l'hydrogène, un groupe alkyle en $C_1$ à $C_6$ ou un groupe répondant à la formule (II):

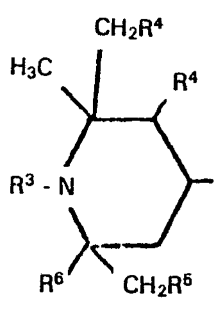

(II)

dans laquelle
$R^3$ est l'hydrogène ou un alkyle en $C_1$ à $C_{18}$,
$R^4$ et $R^5$ soit sont identiques et désignent l'hydrogène, soit désignent un groupe alkyle en $C_1$ à $C_5$, $R^6$ représentant alors un groupe méthyle, ou
$R^4$ étant l'hydrogène ou un groupe alkyle en $C_1$ à $C_5$, et
$R^5$ et $R^6$ représentant avec les atomes de carbone auxquels ils sont liés un noyau cycloalkyle en $C_5$ ou $C_6$ ou un groupe répondant à la formule

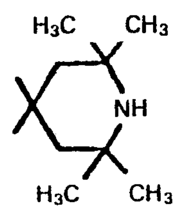

X désigne un groupe répondant à la formule (III)

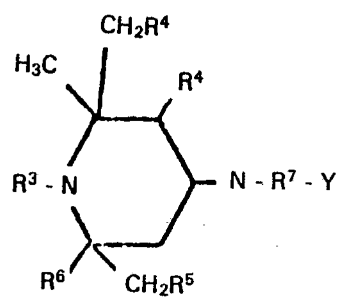

(III)

dans laquelle $R^3$, $R^4$, $R^5$ et $R^6$ ont les significations précédemment indiquées,
$R^7$ représente un groupe alkylène en $C_2$ à $C_4$, qui peut être substitué par un groupe méthyle, et
Y un groupe répondant aux formules $-OR^8$ ou $-N(R^9)_2$ avec $R^8$ = alkyle en $C_1$ à $C_{18}$ et $R^9$ = méthyle ou éthyle, et
$R^1$ lorsque m est un nombre entier de 0 à 3, désigne un groupe alkylène répondant à la formule $-(CH_2)_r$, les indices r et n pouvant être identiques ou différents lorsque m = 0 et désignant un nombre entier de 2 à 6, mais l'indice 1 désignant 2 ou 3 dans le cas où m = 1, 2 ou 3, et les indices r et n étant identiques et désignant également 2 ou 3.

2. Procédé de préparation des composés selon la revendication 1, caractérisé en ce que
A) soit on synthétise à partir de 1 mole d'halogénure de cyanuryle et de 2 moles d'une amine répondant à la formule (VI)

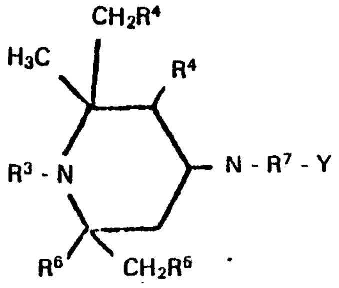

(VI)

dans laquelle $R^3$, $R^4$, $R^5$, $R^6$, $R^7$ et Y ont les significations indiquées dans la revendication 1, en présence de 2 moles d'une base et d'un solvant organique à 10-40°C, un composé répondant à la formule (III)

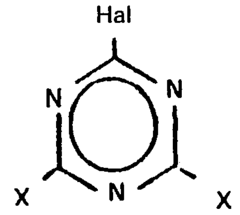

(III)

dans laquelle Hal est l'halogène et X est le radical d'une amine répondant à la formule (VI), après quoi on fait réagir celui-ci dans un solvant organique à une température de réaction de 60 à 150°C avec la quantité équivalente à l'Hal d'une polyamine répondant à la formule (IV)

$$\underset{R^2}{HN}\text{-}R^1-[\underset{H}{N}(CH_2)_l]_m\text{-}\underset{H}{N}(CH_2)_n-\underset{R^2}{NH} \qquad (IV)$$

dans laquelle $R^1$, $R^2$, l, m et n ont les significations indiquées dans la revendication 1, en présence d'une quantité équivalente, par rapport à l'acide halohydrique se formant, d'une base,
B) soit on transforme en dérivé une polyamine (IV) dans un solvant organique inerte à 0-10°C avec la quantité équivalente, par rapport aux fonctions amino, d'un halogénure de cyanuryle en présence de quantités équimolaires, par rapport à la quantité d'acide halohydrique se formant, d'une base, pour donner un composé répondant à la formule (V)

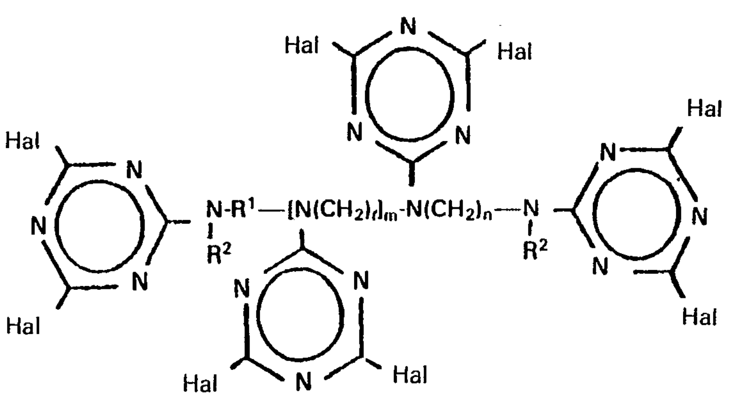

(V)

après quoi on fait réagir celui-ci à 60-150°C dans un solvant organique inerte avec la quantité équivalente, par rapport aux radicaux halogènes, d'un composé répondant à la formule (VI) indiquée ci-dessus, en présence d'une quantité équimolaire, par rapport à l'acide halohydrique qui se forme, d'une base.

3. Procédé selon la revendication 2, caractérisé en ce que les composés intermédiaires (III) ou (V) ne sont pas isolés.

4. Utilisation des composés selon la revendication 1 pour la stabilisation de polymères synthétiques.

5. Utilisation selon la revendication 4, caractérisé en ce que les polymères sont des polyoléfines, des polymères halogénés, des polyacrylates ou des polyméthacrylates ou des homo- ou copolymères du styrène.

6. Procédé pour stabiliser des polymères synthétiques contre les effets destructeurs de la lumière, procédé caractérisé en ce qu'on ajoute aux polymères, le cas échéant en plus de substances connues jusqu'à présent, exerçant une action stabilisante, de 0,01 à 5 parties en poids, par rapport au polymère, d'un stabilisant selon la revendication 1.

**Revendications pour l'Etat Contractant:**
AT

1. Procédé de préparation de composés triaziniques répondant à la formule (I)

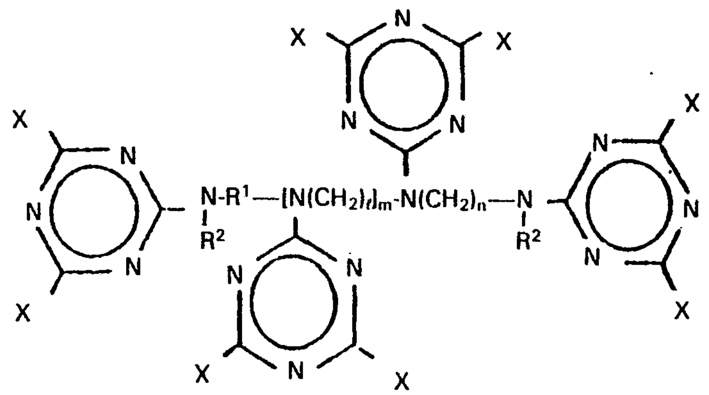

(I)

dans laquelle
$R^2$ désigne l'hydrogène, un groupe alkyle en $C_1$ à $C_6$ ou un groupe répondant à la formule (II):

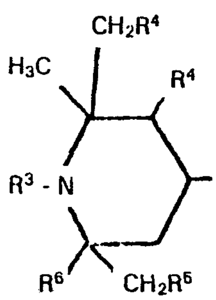

(II)

dans laquelle
$R^3$ est l'hydrogène ou un alkyle en $C_1$ à $C_{18}$,
$R^4$ et $R^5$ soit sont identiques et désignent l'hydrogène, soit désignent un groupe alkyle en $C_1$ à $C_5$, $R^6$ étant alors un groupe méthyle, ou
$R^4$ est l'hydrogène ou un alkyle en $C_1$ à $C_5$, et
$R^5$ et $R^6$ représentent, avec les atomes de carbone auxquels ils sont liés, un noyau cycloalkyle en $C_5$ ou $C_6$ ou un groupe répondant à la formule

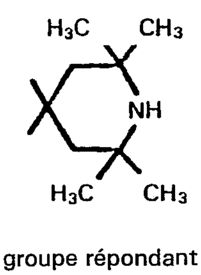

X désigne un groupe répondant à la formule (III)

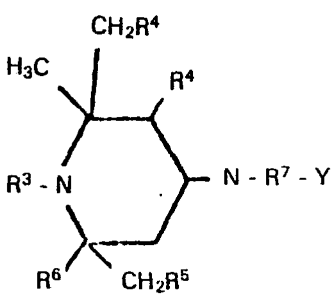

(III)

dans laquelle $R^3$, $R^4$, $R^5$ et $R^6$ ont les significations déjà indiquées,
$R^7$ représente un groupe alkylène en $C_2$ à $C_4$, qui peut être substitué par un groupe méthyle, et
Y un groupe répondant aux formules $-OR^8$ ou $-N(R^9)_2$ avec $R^8$ = alkyle en $C_1$ à $C_{18}$ et $R^9$ = méthyle ou éthyle, et
$R^1$ lorsque m est un nombre entier de 0 à 3, désigne un groupe alkylène répondant à la formule $-(CH_2)_r$, les indices r et n pouvant être identiques ou différents lorsque m = 0 et désignant un nombre entier de 2 à 6, mais l'indice l désignant 2 ou 3 dans le cas où m = 1, 2 ou 3, et les indices r et n étant identiques et désignant également 2 ou 3, caractérisé en ce que
A) soit on synthétise à partir de 1 mole d'halogénure de cyanuryle et de 2 moles d'une amine répondant à la formule (VI)

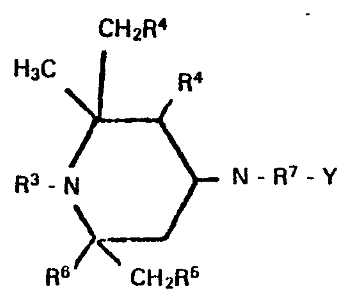

(VI)

dans laquelle $R^3$, $R^4$, $R^5$, $R^6$, $R^7$ et Y ont les significations indiquées dans la revendication 1, en présence de 2 moles d'une base et d'un solvant organique à 10-40°C, un composé répondant à la formule (III)

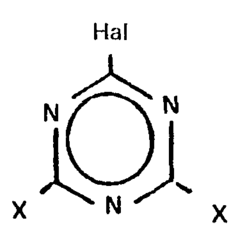

(III)

dans laquelle Hal est l'halogène et X est le radical d'une amine répondant à la formule (VI), après quoi on fait réagir celui-ci dans un solvant organique inerte à une température de réaction de 60 à 150°C avec la quantité équivalente à l'Hal d'une polyamine répondant à la formule (IV)

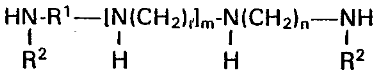

(IV)

dans laquelle $R^1$, $R^2$, l, m et n ont les significations indiquées dans la revendication 1, en présence d'une quantité équivalente, par rapport à l'acide halohydrique se formant, d'une base,

B) soit on transforme en dérivé une polyamine (IV) dans un solvant organique inerte à 0-10°C avec la quantité équivalente, par rapport aux fonctions amino, d'un halogénure de cyanuryle en présence de quantités équimolaires, par rapport à la quantité d'acide halohydrique se formant, d'une base, pour donner un composé répondant à la formule (V)

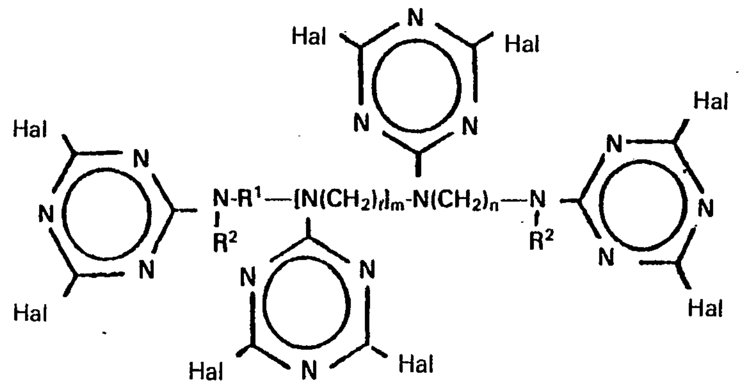

(V)

après quoi on fait réagir celui-ci à 60-150°C dans un solvant organique inerte avec la quantité équivalente, par rapport aux radicaux halogènes, d'un composé répondant à la formule (VI) indiquée ci-dessus, en présence d'une quantité équimolaire, par rapport à l'acide halohydrique qui se forme, d'une base.

2. Procédé suivant la revendication 1, caractérisé en ce que les composés intermédiaires (III) ou (V) ne sont pas isolés.

3. Utilisation des composés préparés suivant l'une quelconque des revendications 1 et 2 pour la stabilisation de polymères synthétiques.

4. Utilisation suivant la revendication 3, caractérisée en ce que les polymères sont des polyoléfines, des polymères halogénés, des polyacrylates ou des polyméthacrylates ou des homo- ou copolymères du styrène.

5. Procédé de stabilisation de polymères synthétiques contre l'influence dégradante de la lumière, caractérisé en ce qu'on ajoute aux polymères, le cas échéant en plus de substances connues jusqu'à présent, ayant une action stabilisante, de 0,01 à 5 parties en poids, par rapport au polymère, d'un stabilisant préparé selon l'une quelconque des revendications 1 et 2.